# EUROPEAN PATENT SPECIFICATION

(11) **EP 3 704 225 B1**
(45) Date of publication and mention of the grant of the patent: **11.10.2023**
(21) Application number: 18870787.1
(22) Date of filing: 29.10.2018
(51) Int. Cl.: C12N 5/07, A61K 9/00, A61K 35/12, A61K 35/28, A61K 35/30

(54) **FETAL TISSUE EXTRACT, METHODS FOR PRODUCING THE EXTRACT, AND THE USE THEREOF**
FETALGEWEBEEXTRAKT, VERFAHREN ZU SEINER HERSTELLUNG UND SEINE VERWENDUNG
EXTRAIT DE TISSUS FOETAUX, PROCÉDÉS DE PRODUCTION DE L'EXTRAIT ET UTILISATION ASSOCIÉE

(30) Priority: 27.10.2017 WO PCT/CN2017/108131
(43) Date of publication of application: 09.09.2020
(73) Proprietor: Alis Pharma Ltd., Central, Hong Kong 999077 (HK)
(72) Inventor: LUI, May Pui-Man, Central Hong Kong 999077 (HK)
(74) Representative: Rüger Abel Patentanwälte PartGmbB
(86) International application number: PCT/CN2018/112444
(87) International publication number: WO 2019/080942

(56) References cited:
- EP-A1- 3 118 307
- EP-A2- 2 277 992
- WO-A1-2009/088314
- WO-A1-2010/007620
- WO-A2-2008/100498
- WO-A2-2011/064669
- XU JIA ET AL: "Porcine brain extract promotes osteogenic differentiation of bone marrow derived mesenchymal stem cells and bone consolidation in a rat distraction osteogenesis model", PLOS ONE, vol. 12, no. 11, 1 November 2017 (2017-11-01), page e0187362, XP055822771, DOI: 10.1371/journal.pone.0187362 Retrieved from the Internet: URL:https://www.ncbi.nlm.nih.gov/pmc/artic les/PMC5665543/pdf/pone.0187362.pdf>
- RAZEGHIAN JAHROMI IMAN ET AL: "The effect of fetal rat brain extract on morphology of bone marrow-derived mesenchymal stem cells", COMPARATIVE CLINICAL PATHOLOGY, SPRINGER-VERLAG, LONDON, vol. 25, no. 2, 5 October 2015 (2015-10-05), pages 343-349, XP036064308, ISSN: 1618-5641, DOI: 10.1007/S00580-015-2188-7 [retrieved on 2015-10-05]
- ZHI-YONG ZHANG ET AL: "Superior Osteogenic Capacity for Bone Tissue Engineering of Fetal Compared with Perinatal and Adult Mesenchymal Stem Cells", STEM CELLS, vol. 27, no. 1, 1 January 2009 (2009-01-01), pages 126-137, XP055099299, ISSN: 1066-5099, DOI: 10.1634/stemcells.2008-0456
- Ramezani M ET AL: "Cell Journal - Ps-102: The Effect of Cartilage Tissue Extraction of 13 Days-Old Mouse Embryo on Differentiation of Bone Marrow Mesenchymal Stem Cells to Osteoblasts |", , 1 January 2013 (2013-01-01), XP055822855, Retrieved from the Internet: URL:https://celljournal.org/journal/articl e/abstract/906 [retrieved on 2021-07-09]
- PHAM TRUC LE-BUU ET AL: "Fetal heart extract facilitates the differentiation of human umbilical cord blood-derived mesenchymal stem cells into heart muscle precursor cells", CYTOTECHNOLOGY, KLUWER ACADEMIC PUBLISHERS, DORDRECHT, NL, vol. 68, no. 4, 7 November 2014 (2014-11-07), pages 645-658, XP036014158, ISSN: 0920-9069, DOI: 10.1007/S10616-014-9812-2 [retrieved on 2014-11-07]
- XU JIA ET AL: "Human fetal mesenchymal stem cell secretome enhances bone consolidation in distraction osteogenesis", STEM CELL RESEARCH & THERAPY, vol. 7, no. 1, 1 December 2016 (2016-12-01), XP055822835, DOI: 10.1186/s13287-016-0392-2 Retrieved from the Internet: URL:https://stemcellres.biomedcentral.com/ track/pdf/10.1186/s13287-016-0392-2.pdf>
- AUGUSTO LÍVIA MARIA MENDONÇA ET AL: "Mesenchymal stromal cells from bone marrow treated with bovine tendon extract acquire the phenotype of mature tenocytes", REVISTA BRASILEIRA DE ORTOPEDIA (ENGLISH EDITION), vol. 51, no. 1, 6 January 2016 (2016-01-06), pages 70-74, XP055822849, ISSN: 2255-4971, DOI: 10.1016/j.rboe.2015.12.013 Retrieved from the Internet: URL:https://www.ncbi.nlm.nih.gov/pmc/artic les/PMC4767843/pdf/main.pdf>
- IN 'TANKER PIETERNELLA S. et al.: "Isolation of Mesenchymal Stem Cells of Fetal or Maternal Origin from Human Placenta", STEM CELLS, vol. 22, no. 7, 29 February 2004 (2004-02-29), pages 1338-1345, XP002581690,

## Description

### FIELD OF THE INVENTION

The present invention relates generally to medical products and the use thereof. The present invention relates more specifically, but not exclusively, to an extract from a fetal or newborn animal for preventing or treating bone disorders or diseases.

### BACKGROUND OF THE INVENTION

It is desirable for people to enhance the quality and span of life. Globally, there are more and more people suffering from bone disorders or diseases. The inherent ability of bone to heal and remodeling (ability to maintain good bone quality) is reduced substantially with aging, thus aging associated bone disorders or diseases is more clinically challenging.

Although diverse medicaments are available on the market for bone disorders or diseases, but their mode of action is primarily targeting on bone resorbing cells (osteoclasts) and bone forming cells (osteoblasts). Emerging evidence suggests that bone, as an endocrine organ, is tightly interacting with various tissues, such as fat tissues, brain and kidney, via systemic biochemical signals in order to maintain the bone formation and resorption and thus bone quality (bone mass, bone structure and bone mechanical strength). Therefore, it is highly desirable in the art to explore new or alternative therapies for preventing or treating bone related disorders or diseases not just via targeting on bone tissues, but via a systemic manner.

ZHI-YONG ZHANG ET AL: "Superior Osteogenic Capacity for Bone Tissue Engineering of Fetal Compared with Perinatal and Adult Mesenchymal Stem Cells", STEM CELLS, vol. 27, no. 1, 1 January 2009 (2009-01-01), pages 126-137, XP055099299, ISSN: 1066-5099, DOI: 10. 1634/stemcells.2008-0456 refers to the proliferative and osteogenic potential of mesenchymal stem cells (MSCs) from human fetal bone marrow (hfMSCs). This document discloses that hfMSCs has the greatest osteogenic differentiation ability when compared with that of human umbilical cord (hUC-MSCs), human adult adipose tissue (hATMSCs) or human adult bone marrow (haMSCs).

Ramezani M. ET AL "The Effect of Cartilage Tissue Extraction of 13 Days-Old Mouse Embryo on Differentiation of Bone Marrow Mesenchymal Stem Cells To Osteoblasts", 1 January 2013 (2013-01-01), XP055822855D9 discusses mouse embryonic cartilage tissue extract. It is noted that the extract of cartilage tissue might have the positive effect on the differentiation of mouse bone marrow derived mesenchymal stem cell into osteoblast in vitro.

XU JIA ET AL: "Human fetal mesenchymal stem cell secretome enhances bone consolidation in distraction osteogenesis", Stem Cell Research & Therapy, vol. 7, no. 1, 1 December 2016 (2016-12-01), XP055822835, DOI: 10.1186/s13287-016-0392-2 refers to human fetal mesenchymal stem cell secretome. This article discloses the secretome was collected from human mesenchymal stem cell (MSC) culture and was used to treat rat bone marrow-derived MSCs (rBMSCs). The secretome can enhance bone consolidation in distraction osteogenesis. That is, D1 also does not discloses any extract derived from any tissue.

### SUMMARY OF THE INVENTION

It is the first time in the art to find that cells or tissue extract derived from animal fetal tissues can effectively prevent or treat bone disorders or diseases.

The invention is defined by the claims. In a first aspect, the present disclosure relates to an extract obtained from a brain or whole fetal tissue of a fetal or newborn animal for use in the prevention or treatment of osteoporosis or bone fracture according to claim 1.

### DESCRIPTION OF THE FIGURES.

Fig 1Workflow in brief.

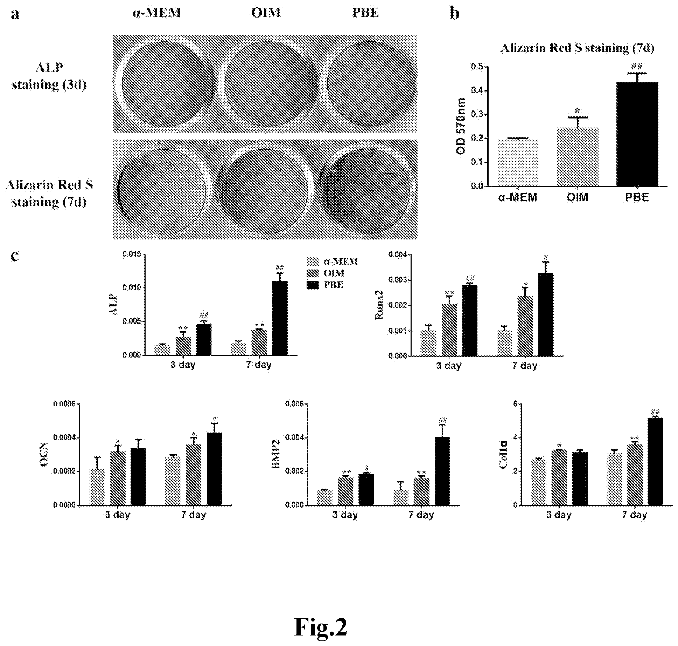

Fig 2. PBE (porcine brain extract) promoted osteogenic differentiation of rat bone marrow derived mesenchymal stem cells (BMSCs) in vitro. (a) The alkaline phosphatase (ALP) staining was conducted after 3-day treatment of PBE. The mineralization potential of rBMSCs was tested by Alizarin Red S staining after 7 days of PBE treatment, (b) Alizarin red S concentrations were quantified by absorbance measurement at 570 nm. (c) The genes expression of osteogenesis-related markers was determined by quantitative real-time PCR after treatment of PBE or osteogenic induction medium (OIM as positive control) for 3 or 7 days. * p < 0.05, ** p < 0.01, compared with the a-MEM group (control); # p < 0.05, ## p < 0.01, compared with the OIM group.
**Fig 3****.** Animal experimental protocol (a) and representative X-rays (b) of distraction regenerate at various time points were present.
**Fig 4****.** PBE treatment improved the quality of new callus as shown by µCT analysis and mechanical test. (a) 3D µCT images of the tibia distraction zone in the two groups at week 3 and 6. (b) The value of BV/TV at week 3 and 6. (c) Mechanical properties (including ultimate load, and energy to failure) of distraction regenerates. *p < 0.05, compared with the phosphate-buffered saline (PBS, control group) group, n=4 at week 3; and n=6 at week 6.
**Fig 5****.** PBE adminstration accelerated new callus consolidation as shown by histological analysis. (a) Representative sections stained with Goldner Trichrome. (b) Von Kossa staining.

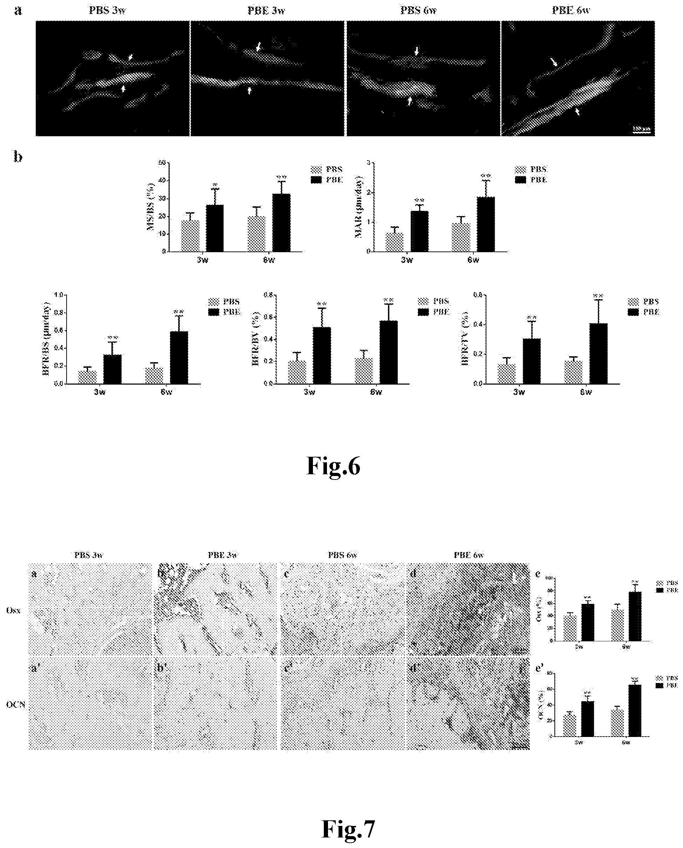

**Fig 6****.** Dynamic histomorphometric measurements showed more quantitative bone formation in the PBE treatment group. (a) Arrows pointed to the calcein (green fluorescent) and xylenol orange (red fluorescent) labeling in representative images of two groups at week 3 and 6. (b) Quantitative measurements of dynamic histomorphometric parameters of (ratio of mineralizing surface to bone surface (MSBS), mineral apposition rate (MAR), bone formation rate per unit of bone surface (BFR/BS), bone formation rate of bone volume (BFR/BV), and bone formation rate of tissue volume (BFR/TV). *p< 0.05, ** p < 0.01, compared with the PBS group.
**Fig 7****.** Representative images of immunohistochemical results of osterix (Osx) (a-e) and osteocalcin (OCN) (a'-e') and quantitative analysis of the positive cells in the distraction regenerates. ** p< 0.01, compared with the PBS group.
**Fig 8****.** Presence of viable CD90+/CD45- cells in PBE, as determined by A) flow cytometry and B) colony forming unit (CFU) assay.
**Fig 9****.** Colony forming unit (CFU) assay indicates the presence of viable cells in fetal sheep tissue extracts. (a and b) colonies derived from viable cells in specified tissues before and after filtration, i.e. the crude (homogenate) and the filtered (filtrate), respectively.
**Fig 10****.** Effect of rat fetal tissue extract on (a) data analysis of fat using DXA; (b) body weight; (c) blood glucose level of fasted blood at the end of treatment period; (d) and (e) data analysis of the mechanical testing (maximal loading and energy to failure) of rat tibia. (f) Data analysis of bone mineral density (BMD) at the fifth vertebra using µCT.
**Fig 11****.** Effect of rat whole fetal extract on the serum level of bone metabolic markers, including osteoprotegerin (OPG), sclerostin (SOST), dickkopf-related protein 1 (DKK1), parathyroid hormone (PTH) and fibroblast growth factor 23 (FGF23), and the serum level of energy metabolic markers, including leptin, amylin and GIP.

### DETAILED DESCRIPTION OF THE INVENTION

Before the present invention is described, it is to be understood that this invention is not limited to particular methods (also referred as "process" hereinafter) and experimental conditions described, as such methods and conditions may vary. It is also to be understood that the terminology used herein is for the purpose of describing particular embodiments only, and is not intended to be limiting, since the scope of the present invention will be limited only by the appended claims.

Unless defined otherwise, all technical and scientific terms used herein have the same meaning as commonly understood by one of ordinary skill in the art to which this invention belongs. As used herein, the term "about," when used in reference to a particular recited numerical value, means that the value may vary from the recited value by no more than 10%.

Although any methods and materials similar or equivalent to those described herein can be used in the practice of the present disclosure, the preferred methods and materials are now described. Other embodiments will become apparent from a review of the ensuing detailed description.

In order that the invention herein described may be fully understood, the following detailed description is set forth.

The present disclosure relates to fetal tissue cells, a fetal tissue extract, an implant containing the extract, a pharmaceutical composition comprising the extract, method from producing the extract, and the medical use of the cells, extract, implant and pharmaceutical composition.

### CELLS FROM TISSUE(S) OF A FETUS OR A NEWBORN ANIMAL

In one aspect, the present disclosure relates to cells from tissue(s) of a fetus or a newborn animal.

"Cell" is the basic structural, functional, and biological unit of all known living organisms. A cell is the smallest unit of life that can replicate independently, and cells are often called the "building blocks of life."

As used herein, a fetus is a stage in the prenatal development of viviparous organisms. "Fetus" refers to different timeframe for different animals. For instance, for a sheep, the fetal stage commences at the beginning of the four week post fertilization. In the context of the present disclosure, a fetal sheep is 12 - 18 week post fertilization, for instance 16 week post fertilization.

In the context of the present disclosure, cells are isolated from tissue(s) of a fetus or a newborn animal.

The amount of cells can be calculated by an automated cell counter or manually by hemocytometer in the unit of number of cells per milliliter.

### EXTRACT FROM TISSUE(S) OF A FETUS OR A NEWBORN ANIMAL

The present disclosure also relates to an extract from tissue(s) of a fetus or a newborn animal.

In a specific embodiment, the extract is a brain extract, for instance, porcine brain extract.

"Extract," "isolate," or "tissue extract" applied interchangeably herein, refer to a complex obtained by extracting a part of a tissue or an organ of a fetus or newborn animal. The commonly used solvents for extract include water or physiological saline such as PBS. In one embodiment of the present disclosure, the solvent is PBS. In one embodiment, the extract comprises live cells from a tissue or an organ of a fetus or newborn animal. In one embodiment, the extract is a cell suspension comprising live cells isolated from a tissue or an organ of a fetus or newborn animal. In another embodiment, the extract substantially contains no cells or contains no cells.

The "tissue" may include but not limited to musculoskeletal tissue, nervous tissue, adipose tissue, epithelial tissue, or umbilical cord tissue, or the combination thereof.

The "organ" may include but not limited to brain, liver, lung, kidney, heart, thymus, spleen, testis, skin, cartilage, thyroid, parathyroid, pancreas, ovaries, eyes, intestinal tract, or tubular organs, or the combination thereof.

In a further embodiment, the extract is a combination of extracts from multiple different tissues. In a specific embodiment, the extract is extract of whole fetus, for instance, a rat whole fetal extract.

"Whole fetal extract," "fetal extract," "extract of fetus," or "tissue extract of fetus" applied interchangeably herein, refer to a complex final product obtained by extracting mixed tissues of a fetus or newborn animal. For instance, a rat whole fetal extract or tissue extract of rat fetus refers to a complex final product obtained by extracting mixed tissues of a rat fetus.

In a further embodiment, the extract retains bioactive components, and/or contains proteins, e.g. 0.1-1000, 0.1-500, 1-200, 10-100, or 70 µg/ml proteins. In a further embodiment, the extract is a filtrate obtained by filtrating and/or centrifuging homogenates of the tissue(s) or organ(s), a processed/purified filtrate obtained by filtrating and/or centrifuging homogenates of the tissue(s) or organ(s), or a fractions of the filtrate or processed/purified filtrate, and wherein the filtrate or the processed/purified filtrate or the fraction preferably retains bioactive components, and/or preferably contains proteins, e.g. 0.1-1000, 0.1-500, 1-200, 10-100, or 70 µg/ml proteins.

The extract can be produced via the following method, comprising the steps of:
a. collecting tissues or organs from the fetal or newborn animal;
b. homogenizing the collected and cut tissues/organs to obtain homogenates;
c. optionally, filtrating and/or centrifuging the homogenates to obtain a filtrate; and
d. optionally, purifying/processing the filtrate further to obtain the final products composed of fetal tissue cells or fetal tissue extract.

In one embodiment, the "filtrating" in step (c) may be performed using sterile filter with desired pore size. For instance, the "filtrating" in step (c) may be performed using a filter which can remove debris. For instance, the pore size of the filter is about 60-80 µm.

In one embodiment, the "purifying" in step (d) may be performed using a filter which can remove viable cells and retain bioactive components. For instance, the filter may be a cell strainer, with the pore size about 0.15-0.30 µm.

In one embodiment, the tissues or organs are collected immediately from the newborn animal following caesarean section.

In a further embodiment, the homogenates are prepared using phosphate-buffered saline.

In a still further embodiment, the centrifugation is performed at 5000g for 15 min at 4°C.

In a still further embodiment, the filtration is performed using a cell strainer. In a yet further embodiment, the extract is kept in liquid nitrogen till use.

In a specific embodiment, the method for producing the extract comprise the steps of:
a. collecting tissues or organs from the fetal or newborn animal as defined;
b. homogenizing the collected tissues to obtain homogenates;
c. filtrating via 70µm filter (also referred to hereinafter as a "strainer") to remove debris and/or centrifuging the homogenates to obtain a filtrate; and
d. purifying/processing the filtrate further through 0.22 µm filter to remove viable cells to obtain the extract.

### ANIMALS

The source or donor animal of the tissues or organs refers to the animal from which the fetal tissues or organs are obtained. The source or donor animal is preferably a mammalian animal, more preferably a sheep, a goat, a pig, a rat, or a mouse.

In one embodiment, the animal is a 12-18 week fetal sheep, for instance, a 16-week fetal sheep. Preferably, the sheep is free of specific pathogen(s).

In another embodiment, the animal is a fetal pig or piglet.

The recipient for the cells or the extract refers to the animal which receives the cells or the extract. The recipient is preferably a mammalian animal. The animal can be any animal suitable for accepting the cells or extract or in need of receiving the cells or extract.

In one embodiment, the recipient is a sheep, a goat, a pig, a rat, or a mouse.

In another embodiment, the recipient is a human.

In further embodiments, the recipient is allogenic or xenogenic to the donor animal. In a specific embodiment, the recipient is xenogenic to the donor animal.

### TYPICAL PROCEDURES FOR PRODUCING THE CELLS OR THE EXTRACT

A typical process for producing the cells or extract of interest may comprise the following procedures:
- animal harvest;
- organ or tissue collection;
- organ or tissue processing; and
- optionally, product testing.

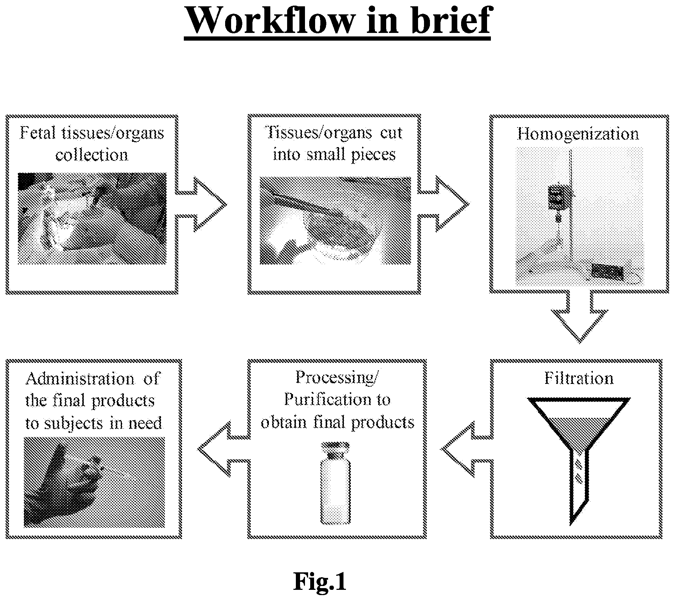

The procedures are shown in Fig. 1 in brief. The procedures may subject to change/amend for optimization, which depend on the purposes of future research studies.

When the donor animal is a fetal sheep free of specific pathogens (an SPF sheep), the "animal harvest" may comprise:
- SPF pregnant sheep transport from SPF Farm to cGMP production area;
- pregnant sheep anesthesia by licensed Vet;
- sheep fetus obtained by licensed Vet by C-section in Animal Operation Theater which is sterile condition.

The "organ or tissue collection" may comprise:
- fetus collected by operating staff, cleaned immediate and being carried to class B production area;
- dissection on sheep fetus to obtain the corresponding organs or tissues;
- organ/tissue washing and being carried to class A laminar flow;
- organ cut/mesh/grind into <0.5cm³ pieces on glass petri dish placing on ice/ice pad.

The "tissue processing" may comprise:
- tissue homogenization by Glas-Col motorized homogenizer at 1600rpm for 30-120sec;
- passing the homogenate through gauze filter;
- filtrate collection for cell count/viability measurement
- product formulation
- product inspection under microscope - to ensure normal cell morphology
- sample archive for future analysis.

Further, the "product testing" method may comprise:
- cell count & viability by automated cell counter, e.g., via automated cell counter TC20 (BioRad) or hemocytometer for manual cell counting;
- microscopic analysis for normal cell morphology.

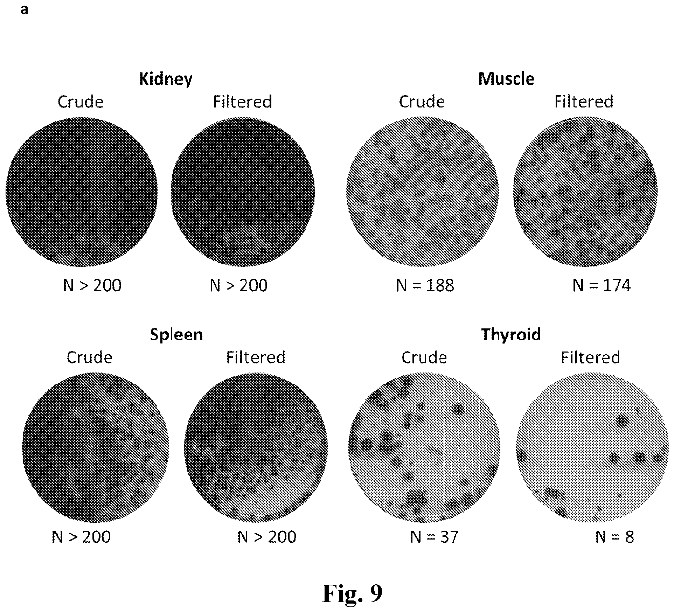

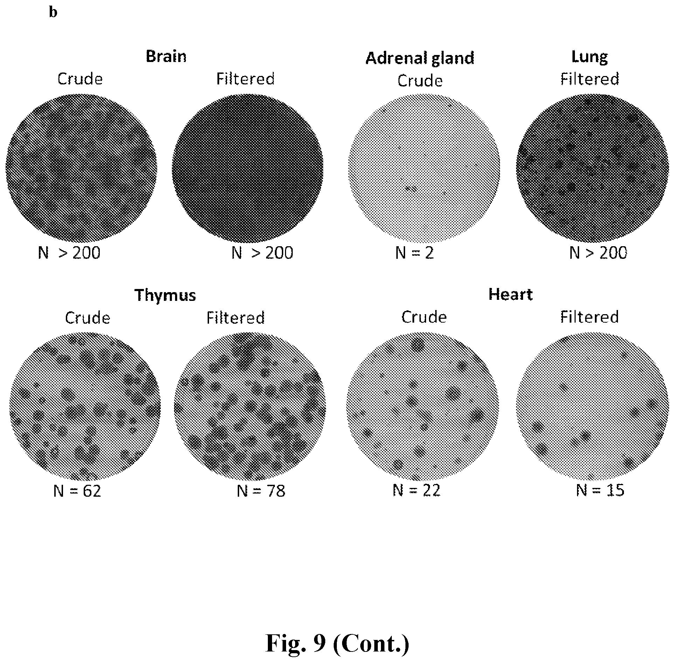

In some embodiments, the cell viability in different tissues are shown in Fig. 9.

### IMPLANT

"Implant" or "fetal tissue implant" interchangeably used herein is implanted into a zone of interest in the recipient, so as to improve the patho-physiological status of the recipient. Specifically, the implant is capable of preventing or treating bone disorders or diseases in the recipient.

In one embodiment, the implant is an allogenic or xenogenic implant, preferably an xenogenic implant.

### PHARMACEUTICAL COMPOSITION

A pharmaceutical composition may comprise the cells of fetal tissues or the fetal tissue extract of the present disclosure, and optionally pharmaceutically acceptable excipients.

Excipients included in the formulations are selected depending on different purposes, e.g., the mode of administration. Examples of generally used excipients included, without limitation: saline, buffered saline, dextrose, water-for-injection, glycerol, ethanol, and combinations thereof, stabilizing agents, solubilizing agents and surfactants, buffers and preservatives, tonicity agents, bulking agents, and lubricating agents.

The amount of the extract to elicit a therapeutic effect can be experimentally determined by a person skilled in the art, depending upon a variety of factors including the age, body weight, general health, sex, and diet of the subject (including, for example, whether the subject is in a fasting or fed state), the time of administration, the rate of excretion, the drug combination, and form of administration. Treatment dosages generally may be titrated to optimize safety and efficacy. Typically, dosage-effect relationships from *in vitro* and/or *in vivo* tests initially can provide useful guidance on the proper doses for subject administration.

In some embodiments, the pharmaceutical composition may be administered to a subject in accordance with known methods, such as intravenous administration, e.g., as a bolus or by continuous infusion over a period of time, by intramuscular, intraperitoneal, intracerebrospinal, subcutaneous, intra-articular, intrasynovial, intrathecal, oral, topical, or inhalation routes.

In another embodiment of the present disclosure, the pharmaceutical composition of the present disclosure is in the form of solid dosage forms, for example tablets (including but not limited to swallowable tablets, chewable tablets, suspension tablets, etc.), capsules, caplets, troches, losenges, powders, granules, *etc.*

### CONDITIONS, DISORDERS AND DISEASES

The cells or extract from a fetal or newborn animal can be used in the prevention or treatment of bone disorders or diseases, and/or enhance bone formation. In some embodiments, bone consolidation/mineralization is enhanced. The bone disorder or diseases comprise but not limited to the condition of low bone mass (such as osteopenia, osteomalacia and osteoporosis), and difficult to heal bone fracture and bone lengthening requiring accelerated bone consolidation. In a specific embodiment, the bone disorder or disease is osteoporosis. Osteoporosis, also known as "porous bones," is a bone disease where increased bone weakness increases the risk of bone fracture. It is the most common reason for a broken bone among the elderly. Osteoporosis becomes more common with age. About 15% of white people in their 50s and 70% of those over 80 are affected. It is more common in women than men. White and Asian people are at the greater risk.

In some embodiments, the cells or the extract from a fetal or newborn animal can also enhance bone formation. In a specific embodiment, the cells or the extract from a fetal or newborn animal can accelerate bone consolidation in distraction osteogenesis.

In some embodiments, the cells or the extract from a fetal or newborn animal can also render beneficial effect on metabolic status such as lower blood glucose and/or fat levels in the recipient.

### EXAMPLES

The following examples are put forth so as to provide those of ordinary skill in the art with a complete disclosure and description of how to make and use the present invention, and are not intended to limit the scope of what the inventors regard as their invention nor are they intended to represent that the experiments below are all or the only experiments performed.

### Example 1: Preparation of Porcine Brain Extract (PBE)

All the chemicals used in the examples were purchased from Sigma-Aldrich, USA, except where specified.

All of the animals were provided by the Laboratory Animal Research Centre of the Chinese University of Hong Kong. All animal experiments were carried out under the animal license issued by the Hong Kong SAR Government and the approval of the Animal Experimentation Ethics Committee of the Chinese University of Hong Kong (Ref NO. 14-052-MIS).

Porcine newborn from around 4-month uncomplicated pregnancy was used for PBE preparation. Neonatal brain tissues were collected immediately from the newborn following caesarean section. The method of euthanasia for the newborn utilized was fast intraperitoneal injection of dorminal 20% with the dosage of 200 mg/kg body weight. After removing the fat tissues, the remaining tissues were washed in ice-cold 0.9% NaCl to remove all traces of blood. The homogenates were then prepared using phosphate-buffered saline (PBS) with a knife homogenizer and polytron homogenizer. The lipids were then removed by filtering through the 70-µm cell strainer, after which the filtrates were collected and centrifuged at 5000g for 15 min at 4 °C, to remove cell debris.

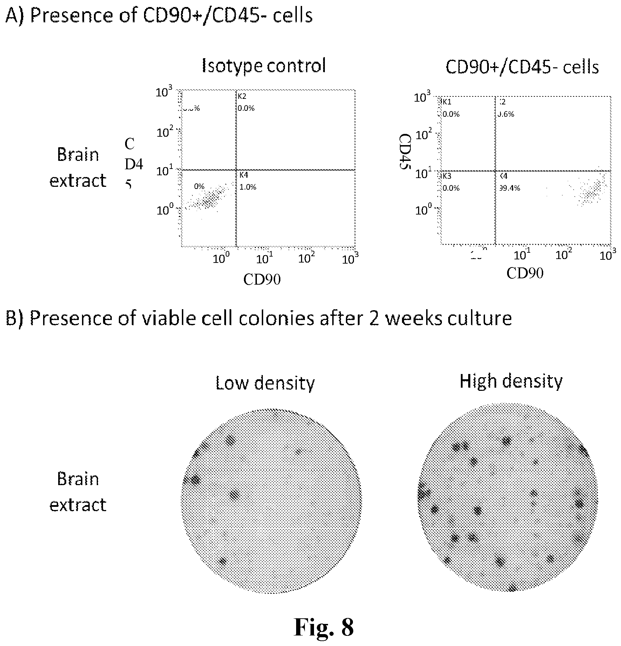

Presence of the cells in the extract were determined via crystal violet staining, and the results are shown in Fig. 8. As shown in Fig. 8, cells were present in the extract.

Finally, the supernatant fluid was further purified using 0.22-µm filters and termed as original PBE. The protein content in the original PBE was measured using BCSA kit (Thermo Scientific, Rockford, IL, USA) according to the manufacturer's instruction, and it was70 µg/ml. The original PBE was kept in liquid nitrogen till its further use. For the cell study, original PBE was diluted 100 times before use to a working concentration of 700 ng/ml, while for animal study, a working concentration of 7 µg/ml (100µl) was used.

### Example 2: Isolation, Culture and Treatment of rBMSCs

Twelve-week-old male Sprague-Dawley (SD) rats were used for rBMSCs (rat bone mesenchymal stem cells) isolation. The method of euthanasia for rats was fast intraperitoneal injection of dorminal 20% with the dosage of 50 mg/kg. Bone marrow was flushed out from the bone cavity of the rats and subject to density gradient centrifugation over Lymphoprep^{™} (1.007 g/ml; AXIS-SHIELD, Norway) to obtain the mononuclear cells (MNCs). The MNCs were cultured in Modified Eagle's Medium of Alpha (α-MEM) (Invitrogen, USA) supplemented with 10% fetal bovine serum (FBS) (Gibco, USA) and 1% penicillin/streptomycin (Gibco, USA) at 37°C with 5% CO2. When colonies were confluent, the cells were trypsinized and re-plated for further expansion and examination. Surface markers including CD31, CD34, CD45, and CD90 (BD Biosciences, USA), were used to determine the purity of MSCs. The rBMSCs used in this study were between passages 3 and 6. [Xu L, Song C, Ni M, Meng F, Xie H, Li G. Cellular retinol-binding protein 1 (CRBP-1) regulates osteogenenesis and adipogenesis of mesenchymal stem cells through inhibiting RXRalpha-induced beta-catenin degradation. The international journal of biochemistry & cell biology. 2012;44:612-619. doi: 10.1016/j.biocel.2011.12.018. PubMed PMID: 22230368.].

The rBMSCs were placed in a 12-well plate at a concentration of 5000 cells/cm² and were incubated in the α-MEM at 37°C in a 95% humidified atmosphere of 5% CO2. When over 80% confluence was reached, the medium was replaced with osteogenic induction medium (OIM) containing 1 nM dexamethasone, 50 uM L-ascorbic acid-2-phosphate, and 20 mM *β-*glycerophosphate or PBE in OIM at a working dose of 700 ng/ml. The OIM and α-MEM only were set as positive and negative control, respectively.

### EXAMPLE 3: PBE promoted osteogenic differentiation of rBMSCs

Fresh PBE and PBE kept in frozen for 2, 4, and 6 weeks (original PBE as prepared in Example 1) were used for testing the effects on osteogenesis of rBMSCs, and no difference on the effects of rBMSCs osteogenesis was found among the various preparation of PBE. To evaluate the effects of PBE on osteogenesis of rBMSCs, ALP and Alizarin Red S staining were performed at day 3 and day 7, respectively.

### Alkaline phosphatase (ALP) staining

After rBMSCs were treated with α-MEM, OIM, and PBE for 3 days, the cells were equilibrated by ALP buffer (0.1 M NaCl, 0.1 M Tris-HCl, 50 mM MgCl₂·6H₂O, PH 9.5) for 5 min twice, incubated with ALP substrate solution (5 µl BCIP and 10 µl NBT in l mL ALP buffer) at 37°C in dark for 60 min, after which the reaction was stopped by distilled water and the plate was dried before taking photos. At day 3 and day 7 of the osteogenic induction with PBE treatment, the genes associated with osteogenesis were assayed by quantitative real-time PCR.

### Alizarin Red S staining

After 7 days of osteogenic induction, rBMSCs were stained with Alizarin Red S (PH 4.2) for 10 min at room temperature and washed with distilled water. To quantify the mineralization, the monolayer was eluted with 10% cetylpyridinium chloride (CPC), and the absorbance was measured at 570 nm.

### RNA extraction and real-time PCR

At day 3 and day 7 of the osteogenic induction with PBE treatment, the genes associated with osteogenesis were assayed by quantitative real-time PCR. Total cellular RNA was isolated with RNA Mini Kit (Invitrogen) according to the manufacturer's instructions. The amount of total RNA reverse-transcribed was 500 ng. First-strand cDNA was synthesized with M-MLV reverse transcriptase (Invitrogen). PCR amplification was performed using Step One Plus Real-Time PCR System (Applied Biosystems, USA). Primer sequences of osteogenic markers were listed in Table 1. The relative quantification of gene expression was analyzed with the values of 2^{-ΔΔCT}, normalized with GAPDH expression level.

**Table 1 Primer sequences for quantitative real-time PCR**

| Gene name | Forward primer sequence (5'-3') Reverse primer sequence (5'-3') | Product Size |
|---|---|---|
| Alkaline phosphatase (ALP) | GGACAATGAGATGCGCCCCACCACCCATGATCACATCG | 101 |
| Bone morphogenetic protein 2 (BMP2) | GCATCGCGCCCCTTATCC GGCGGTACAGGTCGAGCATA | 142 |
| Collagen type 1α(Col1α) | GGAGAGAGCATGACCGA GGGACTTCTTGAGGTTGCCA | 184 |
| Glyceraldehyde-3-phosphate dehydrogenase (GAPDH) | CGGCAAGTTCAACGGCACGAAGACGCCAGTAGACTCCACGAC | 148 |
| Osteocalcin (OCN) | GCATTCTGCCTCTCTGAC(GGGCTCCAAGTCCATTGTT | 133 |
| Runt-related transcription factor 2 (Runx2) | AAGGTTGTAGCCCTCGGATTGAACCTGGCCACTTGGTT | 128 |

### Results

More mineralized nodule formation could be found in PBE group (Fig 2a). The quantitative results showed PBE significantly increased calcium deposition compared to the α-MEM and OIM group (Fig 2b). Furthermore, significant difference in various osteogenic differentiation-related genes was found in the PBE treatment group after osteogenic induction for 3 days and 7 days. The OCN and Col1α in the PBE treated group were significantly upregulated at day 7, but exhibited no difference compared to the OIM group at day 3 (Fig 2c).

### EXAMPLE 4: Animal surgery and distraction protocol

Twenty 12-week-old SD male rats were used. Before surgery, each animal was placed under general anesthesia with a dosage of 0.2 ml/100g body weight via intraperitoneal injection of a solution of 0.2% (vol/vol) xylazine and 1% (vol/vol) ketamine in PBS. Four rats were housed in each cage. All animals were subjected to a right tibia osteotomy procedure with a closed fracture of fibula. A monolateral external distraction fixator (Xinzhong Company, Tianjin, China) was assembled to fix the osteotomy site. Following surgery, rats were allowed to eat and drink ad libitum. Antibiotic (amoxicillin 1.5 mg/100g weight) was administered intraperitoneally for following 3 days. All rats were randomized equally into two groups: PBS group (n = 10): DO with PBS injection; PBE group (n=10): DO with PBE injection. DO: distraction osteogenesis. PBE refers to the original PBE as prepared in Example 1.

The distraction protocol consisted of three phases: latency phase of 5 days, 10-day active distraction phase (1mm/d, in two equal increments), and a consolidation phase of 6 weeks. During the latency and distraction phase, animals were monitored twice a day, while during the consolidation phase, animals were monitored once a day. From the beginning of consolidation phase, both groups received injection of PBS (100 µl) and PBE (100 µl, 7 µg/ml) into the distraction gap every three days till termination, respectively. All rats received subcutaneous injection of Calcein (10 mg/kg) at the beginning of the consolidation phase, and Xylenol Orange (30 mg/kg) three days before termination. No animal became severely ill or died at any time prior to the experimental endpoint. Four rats in each group were terminated at day 36 after surgery, while the rest were terminated at day 57 after surgery. Bilateral tibias were harvested, strapped free of muscle and processed for further examinations.

### EXAMPLE 5: Radiographic assessment of distraction zone

### Digital radiographs

Distraction zone was monitored by weekly X-ray from the beginning of consolidation phase using the digital X-Ray machine (MX-20, Faxitron X-Ray Corp., Wheeling, IL, USA) under an exposure time of 6000 ms and a voltage of 32 kv.

### Micro-computed tomography (µCT)

The structural change within the distraction zone at week 3 and 6 after distraction was quantitatively assessed with a high-solution µCT (µCT40, Scanco Medical, Bassersdorf, Switzerland) [He YX, Zhang G, Pan XH, Liu Z, Zheng LZ, Chan CW, et al. Impaired bone healing pattern in mice with ovariectomy-induced osteoporosis: A drill-hole defect model. Bone. 2011;48:1388-1400. doi: 10.1016/j.bone.2011.03.720. PubMed PMID: 21421090]. Three dimensional (3D) reconstructions of mineralized callus were performed. Histomorphometric analysis was done using sagittal images of the distraction zone. Low- and high- density mineralized callus of distraction zone were reconstructed according to different thresholds (low attenuation = 158, high attenuation = 211) using the established evaluation protocol with small modification [Hao YJ, Zhang G, Wang YS, Qin L, Hung WY, Leung K, et al. Changes of microstructure and mineralized tissue in the middle and late phase of osteoporotic fracture healing in rats. Bone. 2007;41:631-638. doi: 10.1016/j.bone.2007.06.006. PubMed PMID: 17652051]. The high-density tissues (211-1000 threshold) represented the newly formed highly mineralized bone, while the low ones (158-211 threshold) represented the newly formed callus. Bone volume/total tissue volume (BV/TV) of each specimen were recorded for analysis.

### Results

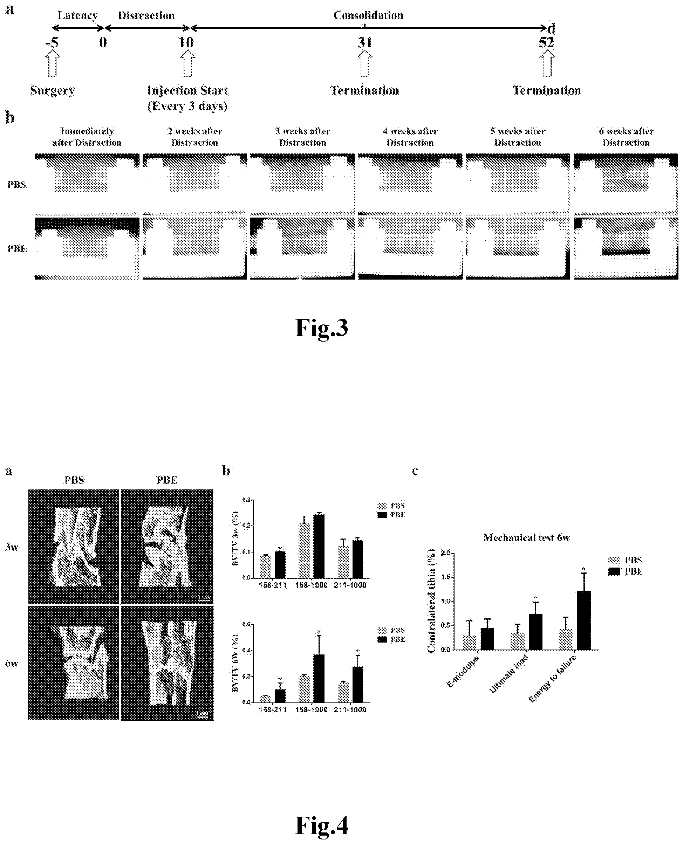

A representative series of X-rays across the time-course of DO show the progression of bone consolidation (Figs 3a and b). Little callus was found in the distraction gap immediately after distraction completed in both groups. As went on, significant more callus was observed in the PBE treatment group compared to the PBS group till termination. Similar results were confirmed by µCT examinations at the 3-week and 6-week (Fig 4a). The value of BV/TV at week 3 was significantly increased in 158-211 threshold, while week 6 significant difference was seen in all three thresholds, indicating more new bone consolidation in the PBE treatment group compared to the PBS group (Fig 4b). PBE refers to the original PBE as prepared in Example 1.

### EXAMPLE 6: Mechanical Testing

### Four-point bending mechanical testing

Specimens harvested at week 6 after distraction were subject to mechanical test within 24 hours after termination. The contralateral tibia was tested as an internal control. A four-point bending device (H25KS; Hounsfield Test Equipment Ltd, Salfords, UK) with a 250N load cell was used to test the tibia to failure. The long axis of tibia was placed perpendicular to the blades during the test [Sun Y, Xu L, Huang S, Hou Y, Liu Y, Chan KM, et al. mir-21 overexpressing mesenchymal stem cells accelerate fracture healing in a rat closed femur fracture model. Biomed Res Int. 2015;2015:412327. doi: 10.1155/2015/412327. PubMed PMID: 25879024; PubMed Central PMCID: PMCPMC4386680]. The modulus of elasticity (E-modulus), ultimate load, and energy to failure were obtained and analyzed using built-in software (QMAT Professional; Tinius Olsen, Inc., Horsham, PA, USA)[Sun Y, Xu L, Huang S, Hou Y, Liu Y, Chan KM, et al. mir-21 overexpressing mesenchymal stem cells accelerate fracture healing in a rat closed femur fracture model. Biomed Res Int. 2015;2015:412327. doi: 10.1155/2015/412327. PubMed PMID: 25879024; PubMed Central PMCID: PMCPMC4386680]. The biomechanical properties of the new bone were expressed as percentages of the contralateral intact bone properties.

### Results

The results of four-point bending mechanical testing in the PBE treatment group at week 6 showed a significant improvement in the ultimate load and energy to failure compared to these of the PBS group after normalized with the contralateral intact tibiae. However, there was no significant difference between both groups in E-modulus (Fig 4c). PBE refers to the original PBE as prepared in Example 1.

### EXAMPLE 7: Histological analysis

### Histology and immunohistochemistry

All specimens were fixed in 10% EDTA formalin for 48 h. Half of them were followed by decalcification in 10% EDTA solution for 3 weeks and embedded into paraffin. 5-µm sections were cut using a rotary microtome (HM 355S, Thermo Fisher Scientific, Inc., Germany) along the long axis in sagittal plane. After deparaffinization, immunohistochemistry staining was done. The other half were managed by gradient alcohol dehydration, xylene defatting, and embedded in methyl methacrylate. Thin (5 µm) and thick (10 µm) sections were cut with the RM2155 hard tissue microtome (Leica, Wetzlar, Germany) along the long axis of distraction zone, respectively. The 5-µm sections were stained with Goldner Trichrome and Von Kossa, while the unstained 10-µm ones were used for dynamic histomorphometric measurements including singled labeled surface (sL.S), double-labeled surface (dL.S), ratio of mineralizing surface to bone surface (MSBS), mineral apposition rate (MAR), bone formation rate per unit of bone surface (BFR/BS), bone formation rate of bone volume (BFR/BV), and bone formation rate of tissue volume (BFR/TV) with fluorescence microscopy (Leica image analysis system, Q500MC) and OsteoMeasure system (OsteoMetrics Inc., Decatur, GA, USA)[Sun Y, Xu J, Xu L, Zhang J, Chan K, Pan X, et al. MiR-503 Promotes Bone Formation in Distraction Osteogenesis through Suppressing Smurf1 Expression. Sci Rep. 2017;7:409. doi: 10.1038/s41598-017-00466-4. PubMed PMID: 28341855; PubMed Central PMCID: PMCPMC5428455].

Immunohistochemistry staining was performed using a standard protocol [ Chen Y, Sun Y, Pan X, Ho K, Li G. Joint distraction attenuates osteoarthritis by reducing secondary inflammation, cartilage degeneration and subchondral bone aberrant change. Osteoarthritis and cartilage / OARS, Osteoarthritis Research Society. 2015;23:1728-1735. doi: 10.1016/j.joca.2015.05.018. PubMed PMID: 26028135]. We incubated paraffin secretions with primary antibodies to rabbit osterix (Osx, Abcam, USA 1:100, ab22552) and osteocalcin (OCN, Santa Cruz, USA 1:100, sc30045) overnight at 4°C. The positive stained cell numbers and area in the whole distraction zone per specimen in three sequential sections (50 µm, 150 µm, and 250 µm) per rat in each group were counted and compared, which were expressed as the percentages of the bone volume.

### Statistical analysis

All quantitative data were analyzed using SPSS 18.0 software for windows (SPSS, Chicago, IL, USA). Mann-Whitney U test with a Bonferroni correction was performed for the comparison of mean values, and P < 0.05 was regarded as statistically significant.

### Results

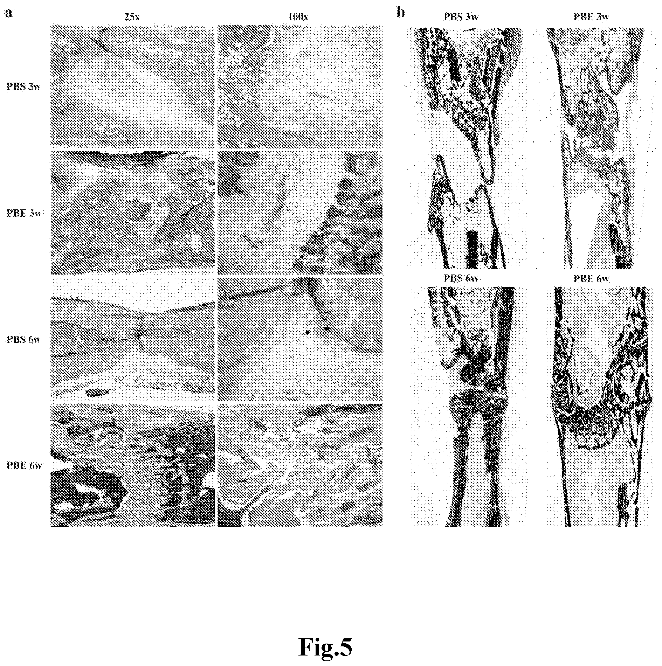

The representative sections from both groups at week 3 and 6 during consolidation phase stained with Goldner Trichrome and Von Kossa were shown in Figs 5a and b. Much more chondrocytes were found in the PBS group than that of the PBE treatment group, especially at week 6, indicating that mineralization of newly formed callus has been accelerated in the PBE treatment group. It was clearly exhibited in the Von Kossa staining that most of new bone had consolidated and the continuity of the cortical bone and bone marrow cavities had almost remodeled in the PBE treatment group at week 6 (Fig 5b). The representative images of dynamic histomorphometric measurements were shown in Fig 6a. The quantitative results demonstrated that the PBE treatment significantly increased MSBS, MAR, BFR/BS, BFR/BV, and BFR/TV, indicating more mineralized bone formation in the PBE treatment group (Fig 6b). The results of immunohistochemistry staining with Osx and OCN revealed a significant increase in the amounts of positive cells in the distraction gap in the PBE treatment group compared to those in the PBS group at week 3 and 6 (Fig 7). All these results demonstrated that PBE treatment enhanced bone formation during DO. PBE refers to the original PBE as prepared in Example 1.

### EXAMPLE 8: Effect of rat fetal extract on bone formation, blood glucose and fat levels

### Materials and Methods

### Animals

Sixteen female Sprague-Dawley rats with 400 grams were obtained from the Laboratory Animal Services Centre of the Chinese University of Hong Kong and were received bilaterally ovarietomy (OVX) operation. OVX rats were then randomized into two groups receiving a 200µl subcutaneous injection of 2mg/ml of rat whole fetal extract (treatment group, n = 8) or phosphate buffer saline (control group, n = 8) and injections were subsequently performed three times a week for three months. Rats were housed in cages at 70°F and constant humidity, with a standard 12:12 h light/dark cycle. Ethics approval was obtained for this animal experiment from the Ethics Committee of the Chinese University of Hong Kong.

Preparation of tissue extract of rat fetus: At day of E12.5, Sprague Dawley (SD) rat fetuses were isolated under dissection microscope. Equal volume of PBS (v/w:1mL/1g) was used as homogenized buffer. Fetuses were homogenized with plastic pastel in a 1.5mL Eppendorf tube (400rpm, 2min) to obtain a homogenate. The homogenate was centrifuged at 5000g for 15 min at 4°C to remove tissue debris, followed by filtration through 70 µm cell strainer and 0.22µm filter to remove cells and debris and to retain bioactive components. The final product, i.e., the rat fetal extract, was stored in -80 °C until use. Total protein concentration was measured by Pierce BCA assay kit (Thermo Fisher USA. Cat. 23225) to standardize the amount for injection.

### Fat

Total body fat mass was measured by using DXA (dual-energy X-ray absorptiometry).

### Body weight

At the day of termination, body weight was measured by Electronic scales for each rat.

### Blood glucose

Briefly, 100µL blood was taken from rat tail. Blood glucose was measured by Bayer contour blood glucose meter at the day of termination. All the rats were fasted overnight before sacrifice.

### Micro-Computed Tomography Imaging Analysis

Micro-computed tomography (MicroCT) was used for quantitative evaluation of the bone formation. The fifth lumbar was scanned by µCT to determine the bone mineral density between control and treatment group as previously described (He, Y.X., et al., Impaired bone healing pattern in mice with ovariectomy-induced osteoporosis: A drill-hole defect model. Bone, 2011. 48(6): p. 1388-400). Briefly, all the specimens were scanned by a µCT (µCT40, Scanco Medical, Bassersdorf, Switzerland) at a custom isotropic resolution of 8 µm isometric voxel size at a voltage of 70 kV with a current of 114 µA.

### Mechanical test

Rat tibia samples were warped with wet gauze and stored at 4°C for no more than 24h before mechanical test at room temperature. A three-point bending device (H25KS; Hounsfield Test Equipment Ltd., UK) with a 250 N load cell was used. During the mechanical testing, the tibias were loaded in the anterior-posterior direction with the inner and outer span of the blades set as 8 and 18 mm, respectively. The long axis of the tibia was placed perpendicular to the blades during the test. After failure of the bone, the following parameters: maximal loading, energy to failure were calculated by built-in software (QMAT Professional; Tinius Olsen, Inc., Horsham, PA, USA).

### Serum metabolic markers

Briefly, 25 µL fasted blood serum was taken from rat tail at the day of termination. Serum level of osteoprotegerin (OPG), sclerostin (SOST), dickkopf-related protein 1 (DKK1), Leptin, parathyroid hormone (PTH), fibroblast growth factor (FGF23), amylin and gastric inhibitory polypeptide (GIP) were quantified with commercial available kits Rat Metabolic Hormone Magnetic Bead Panel - Metabolism Multiplex Assay (EMD Millipore, Billerica, MA, USA) and Rat Bone Magnetic Bead Panel 1 - Bone Metabolism Multiplex Assay (EMD Millipore). The panels were read with Bio-Plex multiplex system (Bio-Plex 200, Bio-Rad Laboratories, Incorporation, CA, USA). The concentrations of the selected analytes were calculated according to the internal reference standard curve for each analyte.

### Results

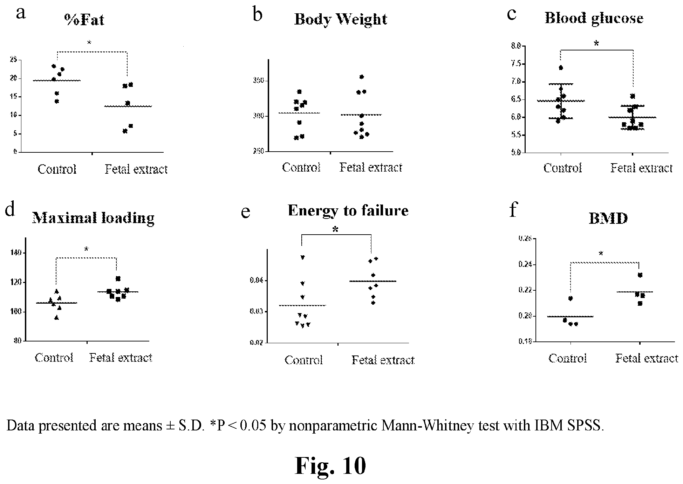

Results on the effects of rat fetal extract on the ratio of the fat mass, body weight, blood glucose and mechanical test are shown in Fig. 10. As shown in Fig. 10 (A), the ratio of the fat mass was significantly decreased in fetal extract treatment group. Fig. 10 (B) demonstrates that body weight showed no difference between two groups while fat ratio changed. Fig. 10 (C) shows that lower blood glucose value was achieved in the treatment group. Fig. 10 (D) and (E) demonstrate that fetal extract treatment group can bear higher force and absorb more energy before fracture. Fig. 10 (F) provides data analysis of BMD (bone mineral density) using µCT. It is shown that the BMD of the metaphysis of the treatment group was higher than that of the control group.

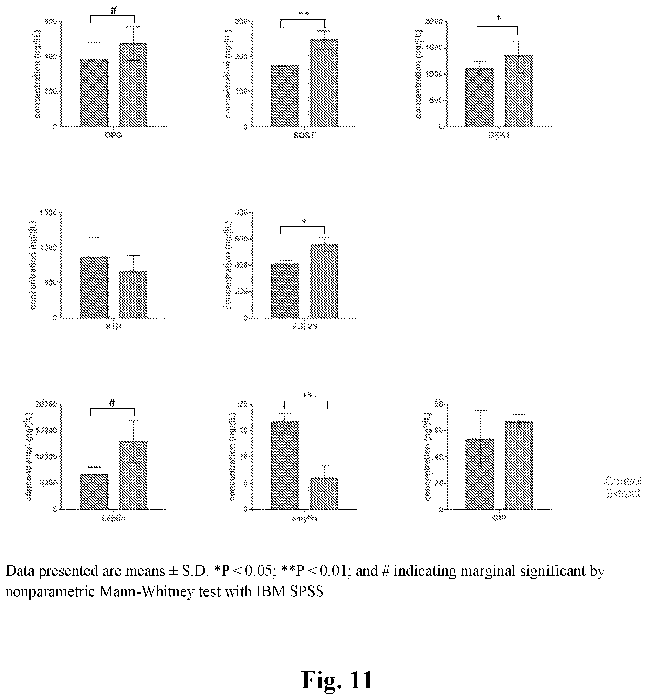

Figure 11 showed the level change of circulatory selective bone and energy metabolic markers in rats after fetal extract treatment in comparison with the untreated control group (OVX only). OPG is a decoy antagonist to RANKL. RANKL is essential factor for osteoclastogenesis, i.e. activation of osteoclast differentiation for bone resorption. Therefore, increase in OPG level might imply less osteoclast differentiation and thus less bone resorption. SOST and DKK1 are key proteins secreted by osteocytes. The increase in these two might imply higher activity of osteocytes which are the most abundant bone cells regulating bone metabolism and mineral homeostasis. PTH is a key bone metabolism related hormone. It regulates SOST level. PTH change is in line with what we observed in SOST. FGF23 secrets osteocytes. The main function is for bone and kidney communication (crosstalk). All these changes are in line with functional and bone histomorphometry study.

Moreover, there was a significant increase in serum leptin level in the treatment group when compared with the control group. Leptin plays a central role in energy regulation. It is correlated with total body fat mass and increases basal metabolic rate leading to weight loss. The increase in leptin might imply higher metabolic activity, physical activity, and decrease appetite. Apart from weight loss, leptin controls directly or indirectly gonadal function by binding to a specific receptor located in the hypothalamus. The leptin receptors can be found in osteoblasts and chondrocytes. It suggests that leptin can induce bone growth and metabolism. The increase in leptin might imply higher activity of hormone secretion or other physiological activity of the gonads. This may result in higher bone mass formation over resorption. Amylin is important control of nutrient fluxes because it reduces energy intake, modulates nutrient utilization by inhibiting postprandial glucagon secretion, and increase energy disposal. The high level of amylin might imply reducing body weight gain and adiposity and increases energy expenditure. The effect of amylin on bone quality is less studied. In vitro studies suggested that amylin could inhibit the fusion of mononucleated osteoclast precursors into multinucleated osteoclasts in an ERK1/2-dependent manner. Nevertheless, the reduction in serum amylin implies direct effect of fetal extract treatment on energy homeostasis. Glucose-dependent insulinotropic polypeptide (GIP) has anabolic effects on bone-derived cells and prevents the bone loss. GIP binding to osteoblastic GIP receptors resulted in an elevation of both cAMP and intracellular calcium. GLP-R leads to an increase in synthesis of collagen type I and increase alkaline phosphatase activity i.e. the bone formation. Thus, increase GIP level might imply higher bone formation.

In summary, the change in these bone metabolic markers are consistent with the Figure 10, indicating significant improvement in bone quality in both histological, mechanical and biochemical levels after fetal extract treatment. On the other hand, significant change in serum energy metabolic markers suggests that the fetal extract treatment could modulate energy homeostasis, which represents an indirect beneficial effect of the invention on bone quality.

## Claims

1. An extract obtained from a brain or whole fetal tissue of a fetal or newborn animal for use in the prevention or treatment of osteoporosis or bone fracture in a human subject, wherein the extract contains no cells, and wherein the extract is xenogeneic to the subject.

2. The extract for use of claim 1, wherein bone mineralization or mechanical property of bone is enhanced.

3. The extract for use of claim 1, wherein the extract is produced by the steps of:
a. collecting brain or whole fetal tissue of a fetal or newborn animal;
b. homogenizing the collected tissues to obtain a homogenate;
c. filtrating and/or centrifuging the homogenate to obtain a filtrate; and
d. purifying the filtrate further to obtain the extract that is an acellular extract.

## Patentansprüche

1. Ein Extrakt, das von einem Gehirn oder einem gesamten Fetalgewebe eines Fötus oder neugeborenen Tieres gewonnen wurde zur Verwendung bei der Vermeidung oder der Behandlung von Osteoporose oder Knochenbrüchen in einem Menschen, wobei das Extrakt keine Zellen enthält und wobei das Extrakt xenogen gegenüber dem Menschen ist.

2. Das Extrakt zur Verwendung nach Anspruch 1, wobei die Knochenmineralisierung oder die mechanische Eigenschaft von Knochen verbessert ist.

3. Das Extrakt zur Verwendung nach Anspruch 1, wobei das Extrakt durch die folgenden Schritte hergestellt wird:
a. Sammeln von Gehirn oder dem gesamten Fetalgewebe eines Fötus oder neugeborenen Tieres;
b. Homogenisieren des gesammelten Gewebes, um ein Homogenat zu erhalten;
c. Filtrieren und/oder Zentrifugieren des Homogenats, um ein Filtrat zu erhalten; und
d. weiteres Reinigen des Filtrats, um das Extrakt zu erhalten, das ein azelluläres Extrakt ist.

## Revendications

1. Extrait obtenu à partir d'un tissu cérébral ou d'un tissu foetal entier appartenant à un foetus animal ou un animal nouveau-né, pour l'utilisation dans la prévention ou le traitement d'une ostéoporose ou d'une fracture osseuse chez un sujet humain, l'extrait ne contenant aucune cellule et l'extrait étant xénogénique pour le sujet.

2. Extrait pour l'utilisation selon la revendication 1, la minéralisation osseuse ou les propriétés mécaniques de l'os étant améliorée(s).

3. Extrait pour l'utilisation selon la revendication 1, étant entendu que l'on produit l'extrait au moyen des étapes suivantes :
(a) prélèvement de tissu cérébral ou de tissu foetal entier sur un foetus animal ou un animal nouveau-né,
(b) homogénéisation des tissus ainsi prélevés, de manière à produire un homogénat,
(c) filtration et/ou centrifugation de l'homogénat de manière à produire un filtrat,
(d) et purification ultérieure du filtrat de manière à produire l'extrait, qui est un extrait acellulaire.
